(19) Europäisches Patentamt
European Patent Office
Office européen des brevets

(11) **EP 3 845 494 A1**

(12) **EUROPEAN PATENT APPLICATION**
published in accordance with Art. 153(4) EPC

(43) Date of publication:
**07.07.2021 Bulletin 2021/27**

(51) Int Cl.:
*C01G 23/04* (2006.01)     *A61K 8/29* (2006.01)
*A61Q 1/12* (2006.01)

(21) Application number: **19855425.5**

(22) Date of filing: **30.08.2019**

(86) International application number:
**PCT/JP2019/034077**

(87) International publication number:
**WO 2020/045617 (05.03.2020 Gazette 2020/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **31.08.2018   JP 2018163676**

(71) Applicant: **Sumitomo Osaka Cement Co., Ltd.**
**Tokyo 102-8465 (JP)**

(72) Inventors:
• **YAKUBO, Teppei**
 **Tokyo 102-8465 (JP)**
• **ITO, Naoko**
 **Tokyo 102-8465 (JP)**

(74) Representative: **Becker, Eberhard**
 **Becker & Kurig Partnerschaft**
 **Patentanwälte PartmbB**
 **Bavariastrasse 7**
 **80336 München (DE)**

(54) **TITANIUM OXIDE POWDER, AND COSMETIC AND DISPERSION LIQUID USING SAME**

(57)     A titanium oxide powder of the present invention contains a polyhedral-shaped titanium oxide particles, in which each particle of the polyhedral-shaped titanium oxide particles has eight or more faces and an average primary particle diameter is 300 nm or higher and 1000 nm or lower, and a crystallinity is 0.95 or higher.

FIG. 1

**Description**

Technical Field

The present invention relates to a titanium oxide powder suitable for cosmetics, and a dispersion and cosmetics using the same.

Priority is claimed on Japanese Patent Application No. 2018-163676, filed on August 31, 2018, the content of which is incorporated herein by reference.

Background Art

Titanium oxide particles have excellent light reflection characteristics, ultraviolet ray shielding characteristics, and opacifying power. Therefore, titanium oxide particles having submicron size to micron size are used for base makeup cosmetics such as foundations.

It is known that the titanium oxide particles used in cosmetics include, for example, octahedral particles in which each particle of the octahedral-shaped particles has line segments each of which connects two apexes facing each other and has a maximum value of the line segments, and an average value of the maximum values is 300 nm or higher and 1000 nm or lower, and a value obtained by dividing the average value of the maximum values by an average particle diameter converted from a BET specific surface area (the average value of the maximum values/a BET-converted average particle diameter) is 1.0 or higher and 2.5 or lower (for example, see Patent Literature No. 1). The titanium oxide particles can be obtained by performing a hydrothermal synthesis reaction as many times.

Reference List

Patent Literature

[Patent Literature No. 1] Pamphlet of International Publication No. WO2018/003851

Summary of Invention

Technical Problem

However, it is considered that in the titanium oxide particles obtained by performing the hydrothermal synthesis reaction as many times, a crystallinity is low, and a refractive index is smaller than a theoretical value. Therefore, in a case where cosmetics containing the titanium oxide particles are applied to the skin, there is a problem that opacifying power is not sufficient.

The present invention has been made in view of the above circumstances, and an object of the present invention is to provide a titanium oxide powder having excellent opacifying power in a case of being applied to the skin, and a dispersion and cosmetics using the same.

Solution to Problem

That is, the titanium oxide powder of the present invention contains polyhedral-shaped titanium oxide particles, in which each particle of the polyhedral-shaped titanium oxide particles has eight or more faces and an average primary particle diameter is 300 nm or higher and 1000 nm or lower, and a crystallinity is 0.95 or higher.

The dispersion of the present invention includes the titanium oxide powder of the present invention and a dispersion medium.

The cosmetics of the present invention contain the titanium oxide powder of the present invention and a cosmetic base.

Advantageous Effects of Invention

According to the titanium oxide powder of the present invention, it is possible to provide a titanium oxide powder having excellent opacifying power in a case of being applied to the skin, and a dispersion and cosmetics using the same.

According to the dispersion of the present invention, in a case where the cosmetics containing this dispersion are applied to the skin, the opacifying power is excellent.

According to the cosmetics of the present invention, in a case of being applied to the skin, the opacifying power is excellent.

Brief Description of Drawings

FIG. 1 is a schematic diagram showing an example of an octahedral-shaped titanium oxide particle.
FIG. 2 is another schematic diagram showing an example of an octahedral-shaped titanium oxide particle.

Description of Embodiments

Embodiments of a titanium oxide powder of the present invention, and a dispersion and cosmetics using the same will be described.
The present embodiments will be described in detail in order to better understand the gist of the invention, and the present invention is not limited unless otherwise specified. The present invention can be changed, omitted, replaced, added, or the like with respect to numerical values, quantities, materials, types, times, temperatures, orders, or the like, without departing from the gist.

[Titanium oxide powder]

A titanium oxide powder of the present embodiment contains polyhedral-shaped titanium oxide particles, in which each particle of the polyhedral-shaped titanium oxide particles has eight or more faces and an average primary particle diameter is 300 nm or higher and 1000 nm or lower, and a crystallinity is 0.95 or higher.
The polyhedral-shaped titanium oxide particles each of which has eight or more faces preferably include octahedral-shaped particles, in which each particle of the octahedral-shaped particles has line segments each of which connects two apexes facing each other and has a maximum value of the line segments, and an average value of the maximum values is 300 nm or higher and 1000 nm or lower.
Hereinafter, "octahedral-shaped particles in which each particle of the octahedral-shaped particles has line segments each of which connects two apexes facing each other and has a maximum value of the line segments, and an average value of the maximum values is 300 nm or higher and 1000 nm or lower" is referred to as "octahedral-shaped particles" in some cases.
The titanium oxide powder of the present embodiment preferably has a diffuse reflectance of 55% or more at 550 nm as measured by the following measuring method.

(Measuring method)

2 g of the titanium oxide powder and 8 g of talc are mixed in a mortar with each other to produce base makeup cosmetics. The base makeup cosmetics are applied on the entirety of one side of a 5 cm square substrate (trade name: HELIOPLATE HD-6, manufactured by Helioscreen) so as to be 12 mg to 14 mg to produce applied substrates.
Next, a diffuse reflection spectrum ($TR_{550\,nm}$) of the coated substrate at 550 nm is measured using a spectrophotometer (model number: UV-3150, manufactured by Shimadzu Corporation). A light reflection spectrum is measured on the basis of a molded plate obtained by compressing barium sulfate powder (special grade, manufactured by Kanto Chemical Co., Inc.).
That is, the titanium oxide powder of the present embodiment preferably contains titanium oxide particles, in which each particle of the titanium oxide particles has eight or more faces and an average primary particle diameter is 300 nm or higher and 1000 nm or lower so that the diffuse reflectance (which may be used as an index of opacifying power) at 550 nm is 55% or more.
Since the diffuse reflectance at 550 nm is 55% or more, the cosmetics containing this titanium oxide powder have excellent opacifying power.
Although a large value of the diffuse reflectance at 550 nm is preferable, the upper limit value may be 100% or less, may be 90% or less, may be 80% or less, may be 70% or less, or may be 65% or less.

(Titanium oxide particle)

The titanium oxide powder of the present embodiment is an agglomerate of titanium oxide particles.
A shape of each titanium oxide particle of the present embodiment is a polyhedral shape with eight or more faces. An average primary particle diameter of the polyhedral-shaped titanium oxide particles each of which has eight or more faces is 300 nm or higher and 1000 nm or lower. The average primary particle diameter is preferably 320 nm or higher and 800 nm or lower, more preferably 340 nm or higher and 700 nm or lower, and even more preferably 400 nm or higher and 600 nm or lower.

Since the shape of each titanium oxide particle is a shape with eight or more faces and the average primary particle diameter is 300 nm or higher and 1000 nm or lower, light can be scattered over a wide range. Therefore, when the cosmetics containing the titanium oxide powder are applied to the skin, a feeling of transparency and opacifying power can be improved.

Here, the primary particle diameter of the titanium oxide particle means the length of the longest linear portion of the titanium oxide particle (longest diameter). The linear portion is not a surface of the particle but may be within the inside of the particle. For example, a primary particle diameter of a spherical titanium oxide particle means diameter. In another example, a primary particle diameter of a rod-shaped titanium oxide particle approximately means the longest linear portion in a longitudinal direction. For example, the primary particle diameter of the octahedral-shaped titanium oxide particle means the maximum value of line segments in one particle, each of which connects two apexes facing each other (hereinafter, may be referred to as a "distance between apexes"). The two apexes facing each other are not adjacent to each other. That is, in the two apexes, the line segment connecting the apexes to each other is a line segment that does not pass through the surface of the particle but passes through the inside of the particle. The maximum value is obtained by a combination of apexes that are farthest from each other.

The primary particle diameter of the titanium oxide particle is determined by the following method. In a case where the titanium oxide powder of the present embodiment is observed using a scanning electron microscope (SEM), a predetermined number of titanium oxide particles, for example, 200 particles, 100 particles, or 50 particles are selected. Then, the primary particle diameter can be obtained by measuring the longest linear portion (longest diameter) of each of these titanium oxide particles.

In a case where the titanium oxide particles are agglomerated with each other, particle diameters of the agglomerated particles in this agglomerate is not measured. The titanium oxide particles (primary particles) constituting this agglomerate are measured and used as the primary particle diameters.

In a case where a shape of the titanium oxide particle is broken and the shape before damaged can be presumed, the primary particle diameter is measured using the shape of the particle before damaged.

The average primary particle diameter in the present embodiment means a value in a case where a cumulative volume percentage of a volume particle diameter distribution of the primary particle diameter of the titanium oxide particle is 50%.

d50 is obtained by, for example, the following order.

The primary particle diameter of 100 titanium oxide particles is measured. The measured primary particle diameter is cubed and multiplied by a constant to obtain a volume. The constant may be appropriately determined according to the shape of the titanium oxide particle. For example, a constant for the octahedral-shaped particle is 0.05, and a constant for the spherical particle is 0.52 ($4\pi/3/8$). The measured primary particle diameter and the calculated volume value are used to calculate the volume particle diameter distribution of the primary particle diameter. The average primary particle diameter d50 means the primary particle diameter in a case of cumulative 50%.

An amount of the polyhedral-shaped titanium oxide particles, each of which has eight or more faces, in the titanium oxide powder is represented by % by number calculated by a method described later. That is, the amount of the polyhedral-shaped titanium oxide particles in the titanium oxide powder, each of which has eight or more faces, in the titanium oxide powder is preferably 50% by number or more, and may be 60% by number or more, or may be 70% by number or more. The upper limit of an amount of the polyhedral-shaped titanium oxide particles, each of which has eight or more faces, in the titanium oxide powder may be 70% by number, may be 80% by number, may be 90% by number, or may be 100% by number.

A case where the amount of the polyhedral-shaped titanium oxide particles, each of which has eight or more faces, in the titanium oxide powder is 50% by number or more is advantageous from the viewpoint of further decreasing paleness peculiar to titanium oxide particles while having excellent opacifying power and a feeling of transparency in a case where cosmetics containing the titanium oxide powder are applied to the skin.

The amount of the polyhedral-shaped titanium oxide particles, each of which has eight or more faces, in the titanium oxide powder of the present embodiment, that is, % by number can be calculated by, for example, observing 100 titanium oxide particles contained in the titanium oxide powder with a scanning electron microscope (SEM) and counting the number of polyhedral-shaped titanium oxide particles each of which has eight or more faces, and which are included in the 100 particles.

Any polyhedral shape with eight or more faces can be selected. A particle having a polyhedral shape is a particle with a plurality of faces. Examples of the polyhedral shape include an octahedral shape, a decahedral shape, a dodecahedral shape, an icosahedral shape, and a star shape. Each face of the polyhedral shape may be substantially the same, or may include faces having a plurality of different shapes from each other, such as two types. The polyhedral shape may be a regular polyhedral shape or may be another polyhedral shape. Specific examples of the polyhedral shape include shapes such as a regular octahedron and a rectangular bipyramid. Among these, an octahedral shape is preferable from the viewpoint that light can be scattered over a wide range.

The polyhedral shape also includes a shape in which corners of the polyhedron are rounded and the entirety or a part of the polyhedron is rounded.

The polyhedral shape also includes a shape in which a part of a particle having the polyhedral shape is damaged. That is, in a case where a particle has a shape similar to the polyhedral-shaped particle or is presumed to have the shape formed from the polyhedral-shaped particle due to the breakage, the particle is regarded as the polyhedral-shaped particle.

In addition, the polyhedral shape also includes an agglomerated particle in which polyhedral-shaped particles are agglomerated.

Hereinafter, octahedral-shaped titanium oxide particles will be described in detail as a preferred example of the present embodiment.

A shape of each of the titanium oxide particles in the present embodiment is an octahedral shape in which an average value of the maximum values of the line segments each of which connects two apexes facing each other is 300 nm or higher and 1000 nm or lower. The maximum value of the line segments of the octahedral-shaped particle, each of which connects the two apexes facing each other, corresponds to the primary particle diameter.

Since the shape of the titanium oxide particle is the octahedral shape in which the average value of the maximum values of the line segments each of which connects two apexes facing each other is 300 nm or higher and 1000 nm or lower, it is possible to scatter light over a wide range. Therefore, opacifying power and a feeling of transparency can be improved in a case where the cosmetics containing the titanium oxide powder are applied to the skin.

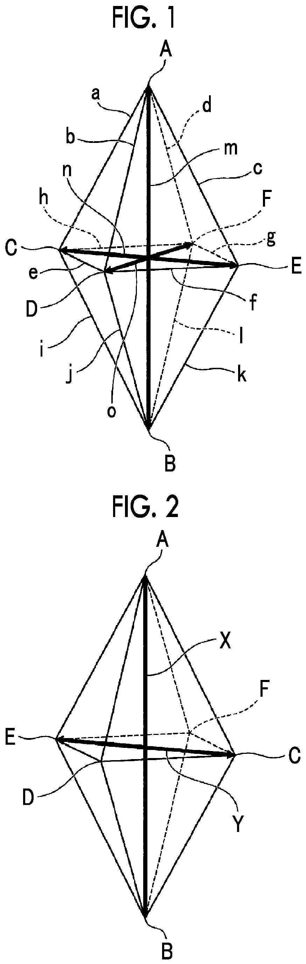

The octahedral shape is a three-dimensional shape having a space surrounded by eight triangles, as shown in FIG. 1.

A tip end part of each apex of the octahedral-shaped titanium oxide particle (points indicated by reference signs A, B, C, D, E, and F in FIG. 1) may have a sharp shape, a rounded shape, or a flat shape. Corners of the octahedron are rounded and the entirety or a part of the octahedron is rounded. The octahedral-shaped titanium oxide particle also has a shape in which a part of the octahedral-shaped particle is damaged. That is, in a case where a particle has a shape similar to the octahedral-shaped particle or is presumed to have the shape formed from the octahedral-shaped particle due to the breakage, the particle is regarded as the octahedral-shaped particle.

In addition, the octahedral-shaped titanium oxide particles may be agglomerated particles obtained from which the octahedral-shaped titanium oxide particles are agglomerated with each other.

An amount of the octahedral-shaped titanium oxide particles in the titanium oxide powder, in which an average value of the maximum values of the line segments each of which connects two apexes facing each other is 300 nm or higher and 1000 nm or lower, is represented by % by number calculated by a method described later. The amount of the octahedral-shaped titanium oxide particles in the titanium oxide powder is preferably 50% by number or more, may be 60% by number or more, or may be 70% by number or more. The upper limit of the amount of the octahedral-shaped titanium oxide particles in the titanium oxide powder may be 80% by number, may be 90% by number, or may be 100% by number.

A case where the amount of the octahedral-shaped titanium oxide particles in the titanium oxide powder is 50% by number or more is preferable from the viewpoint of further decreasing paleness peculiar to titanium oxide particles while having excellent opacifying power and a feeling of transparency in a case where cosmetics containing titanium oxide powder are applied to the skin.

The amount of the octahedral-shaped titanium oxide particles in the titanium oxide powder, that is, % by number can be calculated by, for example, observing 100 titanium oxide particles with a scanning electron microscope (SEM) and counting the number of octahedral-shaped titanium oxide particles, which are included in the 100 particles.

(Line segment connecting two apexes facing each other)

Each particle of the octahedral-shaped particles has line segments each of which connects two apexes facing each other (hereinafter, referred to as "distances between apexes" in some cases) and has a maximum value of the line segments, and an average value of the maximum values is 300 nm or higher and 1000 nm or lower, preferably 320 nm or higher and 800 nm or lower, more preferably 340 nm or higher and 700 nm or lower, and even more preferably 400 nm or higher and 600 nm or lower.

The octahedral-shaped particles, in which each particle of the octahedral-shaped particles has distances between two apexes facing each other and has a maximum value of the distances, and an average value of the maximum values is 300 nm or higher and 1000 nm or lower, is capable of scattering visible light over a wide range, as compared with sphericalshaped titanium oxide particles and spindle-shaped titanium oxide particles. Therefore, it is presumed that cosmetics that contain a titanium oxide powder including the octahedral-shaped particles are capable of decreasing paleness peculiar to titanium oxide particles while achieving both opacifying power and a feeling of transparency.

A case where the octahedral-shaped particles each of which has distances between two apexes facing each other and has a maximum value of the distances and in which an average value of the maximum values is 300 nm or higher and 1000 nm or lower is advantageous from the viewpoint of further decreasing paleness peculiar to titanium oxide particles while having an excellent feeling of transparency in a case of being applied to the skin.

In a case where each particle of the octahedral-shaped particles has an average value of the maximum values of the distances between two apexes facing each other lower than 300 nm, light having a short wavelength is scattered. Therefore, a pale color is given, which is not preferable. On the other hand, in a case where each particle of the octahedral-shaped particles has an average value of the maximum values of the distances between two apexes facing each other in one particle higher than 1000 nm, a feeling of transparency is not obtained, which is not preferable.

The maximum value of distances between two apexes facing each other in the octahedral-shaped particle is measured by observing the octahedral-shaped particles with a scanning electron microscope (SEM). Specifically, with respect to 100 octahedral-shaped particles, each of the maximum values of the distances between two apexes facing each other is measured. The volume average (d50) of the obtained measured values is an average value of the maximum values of the distances between the two apexes facing each other. A method for measuring d50 is the same as described above.

In a case where tip end parts of the apexes of the octahedral-shaped particle are flat surfaces, the maximum values of the distances between two apexes facing each other are measured by using center points of the flat surfaces as the apexes.

In the octahedral-shaped particle, the maximum value of lengths of the line segments (long axis m of the octahedral-shaped particle shown in FIG. 1) each of which connects two apexes facing each other (points A and B shown in FIG. 1) is denoted by X (nm). In the octahedral-shaped particle, the minimum value of lengths of the line segments (short axises n and o of the octahedral-shaped particle shown in FIG. 1) substantially orthogonal to the line segment (long axis m of the octahedral-shaped particle shown in FIG. 1) corresponding to the maximum value and each of which connects two apexes facing each other (points C and E or points D and F shown in FIG. 1) is denoted by Y (nm). In this case, an arithmetic mean value of ratios of X to Y (X/Y) is preferably 1.5 or higher and 3.0 or lower, and more preferably 1.5 or higher and 2.5 or lower.

A case where the average value of the ratio (X/Y) is 1.5 or higher and 3.0 or lower is advantageous from the viewpoint that cosmetics containing a titanium oxide powder including the octahedral-shaped particles can obtain a light scattering effect of the octahedral-shaped particles in a more effective manner and can further improve a feeling of transparency in a case of being applied to the skin.

"substantially orthogonal" as described above indicates that two line segments (the long axis and the short axis of the octahedral-shaped particle) intersect at an angle of 70° to 90°. In addition, regarding "substantially orthogonal" as described above, two line segments (the long axis and the short axis of the octahedral-shaped particle) may be close to and intersect each other, or two line segments (the long axis and the short axis of the octahedral-shaped particle) may not necessarily have an intersection point.

The octahedral shape is a rectangular bipyramidal shape in which two rectangular pyramids share a rectangular bottom surface. The octahedral shape in the present embodiment is preferably a shape in which two congruent rectangular pyramids share a square bottom surface. In addition, in the present embodiment, a side surface shape of the rectangular pyramid is an isosceles triangle, not an equilateral triangle. The maximum value (X) of distances between two apexes facing each other in the octahedral-shaped particle means a length of the line segment that gives a distance between two apexes present in a direction orthogonal to the bottom surface of the rectangular pyramids. In addition, the minimum value (Y) of distances between two apexes facing each other in the octahedral-shaped particle means a length of a shorter diagonal line in two diagonal lines on the bottom surface of the two rectangular pyramids.

Here, the distances between two apexes will be described with reference to the drawings. FIG. 1 is a schematic diagram showing an example of the octahedral-shaped titanium oxide particle in the titanium oxide particles of the present embodiment. As distances between two apexes in the octahedral-shaped particle, there are 15 distances of a distance a between points A and C, a distance b between points A and D, a distance c between points A and E, a distance d between points A and F, a distance e between points C and D, a distance f between the points D and E, a distance g between points E and F, a distance h between points F and C, a distance i between points B and C, a distance j between points B and D, a distance k between points B and E, a distance 1 between points B and F, a distance n between points C and E, a distance o between points D and F, and a distance m between points A and B, as shown in FIG. 1. In addition, a, b, c, d, e, f, g, h, i, j, k, 1 in the figure may mean each side in addition to the above described distances. m, n, o may mean each line connecting the facing apexes in the particle in addition to the above described distances. In FIG. 1, as distances between two apexes facing each other in the octahedral-shaped particle, there are 3 distances of the distance n between points C and E, the distance o between points D and F, and the distance m between points A and B. A maximum value of the distances between two apexes facing each other in the octahedral-shaped particle is the distance m that corresponds to the maximum value (X) of the distances between two apexes facing each other in the octahedral-shaped particle. In addition, in FIG. 1, the line segments each of which is substantially orthogonal to the line segment (line segment X) corresponding to the maximum value X and each of which connects other two apexes facing each other in the octahedral-shaped particle are the distance n and the distance o. In the distance n and the distance o, a shorter distance corresponds to the minimum value (Y) of the distances between two apexes facing each other in the octahedral-shaped particle. A line segment corresponding to the minimum value (Y) may be considered as a line segment Y.

The maximum value (X) (nm) of the distances between two apexes facing each other in the octahedral-shaped particle and the minimum value (Y) (nm) of the distances between two apexes facing each other in the octahedral-shaped particle can be measured, for example, by observing the octahedral-shaped particles with a scanning electron microscope (SEM).

In a case where a part of the octahedral-shaped particle is damaged and the shape before damaged can be presumed, the above maximum value (X) (nm) and the minimum value (Y) (nm) are measured using the shape of the octahedral-shaped particle before damaged. In a case octahedral-shaped particles are agglomerated, the shape of one octahedral-shaped particle in the agglomerated particles is presumed to measure the above maximum value (X) (nm) and the minimum value (Y) (nm).

The above ratio (X/Y) is calculated by observing the titanium oxide particles with a scanning electron microscope (SEM) and measuring the maximum value (X) and the minimum value (Y). The value obtained by calculating the ratio (X/Y) of each particle for the 100 octahedral-shaped titanium oxide particles and calculating an arithmetic mean of the obtained values is an average value of the above ratios (X/Y).

(Crystallinity)

The titanium oxide powder of the present embodiment has a crystallinity of 0.95 or higher, preferably 0.96 or higher, more preferably 0.97 or higher, and even more preferably 0.98 or higher. The upper limit of the crystallinity of the titanium oxide powder of the present embodiment is 1.0.

Since the crystallinity is 0.95 or higher, a refractive index of the titanium oxide particles is increased, light scattering intensity is increased, and light can be scattered with a small amount of addition. Therefore, in a case where the titanium oxide powder is mixed in cosmetics, opacifying power and a feeling of transparency are excellent.

The crystallinity of the titanium oxide powder can be measured by X-ray diffraction (XRD: X-ray diffraction). Specifically, for example, first, X-ray intensity is measured in an output of 45 kV and 40 mA at a diffraction angle 2θ in a range of 20° to 30° using CuKα ray as an X-ray source with an X-ray diffractometer (trade name: X'Pert PRO MPS, manufactured by Malvern Panalytical Ltd.). The obtained X-ray diffraction pattern is profile-fitted for a crystalline portion (peak) and an amorphous portion (halo), and integrated intensities of each are calculated. A ratio of the integrated intensities of the crystalline portion to a total integrated intensity is defined as a crystallinity.

The crystallinity may be determined using the following expression.

```
Crystallinity = integrated intensity derived from
crystals/(integrated intensity derived from crystals +
integrated intensity derived from amorphous)
```

In other words, the crystallinity may be determined using the following expression.

```
Crystallinity = peak area of crystal component/(peak
area of crystal component + halo pattern area of amorphous
component)
```

(Crystalline phase)

A crystalline phase of the titanium oxide powder of the present embodiment is not particularly limited, and may be any one single phase of an anatase type, a rutile type, or a brookite type, or may be a mixed phase thereof. Among these, the crystalline phase of the titanium oxide powder of the present embodiment preferably has the anatase type. A case where the crystalline phase of the titanium oxide powder is the anatase type is advantageous from the viewpoint that opacifying power is further increased in a case where cosmetics containing the titanium oxide powder are applied to the skin, and a color which is close to a color of the human skin is obtained in a case of being mixed with a cosmetic base.

The fact that the titanium oxide powder has the anatase type can be confirmed by, for example, an X-ray diffractometer (trade name: X'Pert PRO, manufactured by Spectris Co., Ltd.). In a case where a measurement result obtained by the X-ray diffractometer is the anatase single phase, a titanium oxide powder has the anatase type.

(Specific surface area)

A BET specific surface area of the titanium oxide powder of the present embodiment is preferably 5 $m^2/g$ or higher and 15 $m^2/g$ or lower, and more preferably 5 $m^2/g$ or higher and 13 $m^2/g$ or lower.

A case where the BET specific surface area of the titanium oxide powder is 5 $m^2/g$ or higher and 15 $m^2/g$ or lower is advantageous from the viewpoint of further decreasing paleness peculiar to titanium oxide particles while having opacifying power and a feeling of transparency. Furthermore, in a case where the BET specific surface area of the titanium oxide powder is lower than 5 $m^2/g$, a feeling of transparency is deteriorated due to light scattering. On the other hand, in a case where the BET specific surface area of the titanium oxide powder is higher than 15 $m^2/g$, scattering intensity of light having a short wavelength increases as compared with scattering intensity of light having a long wavelength, and thus paleness increases.

As a method for measuring the BET specific surface area, for example, a method for performing measurement from a nitrogen adsorption isotherm by the BET multipoint method using a fully automated specific surface area measuring device (trade name: BELSORP-Mini II, manufactured by MicrotracBEL Corp.) is mentioned.

(Average particle diameter converted from BET specific surface area)

An average particle diameter (hereinafter also referred to as "BET-converted average particle diameter") of the titanium oxide powder which is converted from a BET specific surface area of the titanium oxide powder is preferably 300 nm or higher and 1000 nm or lower, more preferably 310 nm or higher and 800 nm or lower, and even more preferably 320 nm or higher and 700 nm or lower.

The BET-converted average particle diameter of the titanium oxide powder can be calculated by Expression (1) in a case where the titanium oxide particles have an octahedral shape.

$$\texttt{BET-converted average particle diameter (nm) =}$$

$$\texttt{16240/(BET specific surface area (m}^2\texttt{/g)} \times \rho \texttt{ (g/cm}^3\texttt{)) ... (1)}$$

In Expression (1), $\rho$ represents a density of the titanium oxide.

Even in a case where the titanium oxide powder contains titanium oxide particles having a shape other than octahedral-shaped particles, the BET-converted average particle diameter is calculated by using Expression (1) in a case where the octahedral-shaped particles of 50% by number or more are contained in the titanium oxide powder.

(Average value of maximum values/BET-converted average particle diameter)

A value obtained by dividing the average value of the maximum values of the line segments each of which connects the two apexes by the average particle diameter of the octahedral-shaped particles, which is converted from the BET specific surface area (the average value of the maximum values/the BET-converted average particle diameter), is preferably 0.5 or higher and 2.5 or lower, more preferably 0.7 or higher and 1.4 or lower, and even more preferably 0.9 or higher and 1.3 or lower.

In a case where (the average value of the maximum values/the BET-converted average particle diameter) is lower than 0.5, it is assumed that fine pores and the like are present in the titanium oxide particles, a refractive index as particles is lower than an original value of titanium oxide particles, which may, as a result, decrease opacifying power. On the other hand, in a case where (the average value of the maximum values/the BET-converted average particle diameter) is higher than 2.5 and cosmetics containing the titanium oxide powder are applied to the skin, a light scattering effect due to a shape of the titanium oxide particle cannot be obtained, and a feeling of transparency cannot be improved.

In general, in a case where the octahedral-shaped particles do not agglomerate with one another, the average particle diameter of the octahedral-shaped particles, which is converted from the BET specific surface area, roughly matches the average value of the maximum values of the line segments, each of which connects two apexes facing each other in the octahedral-shaped particle and is measured by making an observation with an electron microscope.

Therefore, the fact that the value of (the average value of the maximum values/the BET-converted average particle diameter) is closer to 1.0 means that the titanium oxide particles are less likely to agglomerate with one another and more particles are present in a state of primary particles.

On the other hand, in a case where primary particles agglomerate with one another to form the octahedral-shaped particles, the average value of the maximum values of the line segments, each of which connects two apexes facing each other in the octahedral-shaped particle and is measured by making an observation with an electron microscope, does not match the average particle diameter of the octahedral-shaped particles converted from the BET specific surface area. Therefore, in a case where the primary particles agglomerate with one another to form the octahedral-shaped particles, the average value of the maximum values/the BET-converted average particle diameter is higher than 2.5.

(Surface treatment)

The titanium oxide powder of the present embodiment may have any of an inorganic compound or an organic compound on a surface thereof.

Examples of a method for attaching any of the inorganic compound or the organic compound to the surface of the titanium oxide particles include a method for performing a surface treatment using a surface treatment agent, and the like.

The surface treatment agent is not particularly limited as long as the surface treatment agent can be used in cosmetics, and can be appropriately selected depending on a purpose. Examples of the surface treatment agent include an inorganic component, an organic component, and the like.

Examples of the inorganic component include inorganic oxides such as silica and alumina.

Examples of the organic component include a silicone compound, a fatty acid, fatty acid soap, fatty acid ester, an organic titanate compound, a surfactant, a non-silicone compound, and the like. One of these may be used alone, or two or more thereof may be used in combination.

Examples of the silicone compound include silicone oil, alkylsilane, fluoroalkylsilane, methicone, hydrogendimethicone, triethoxysilylethyl polydimethylsiloxyethyl dimethicone, triethoxysilylethyl polydimethylsiloxyethyl hexyl dimethicone, (acrylates/tridecyl acrylate/triethoxysilylpropyl methacrylate/dimethicone methacrylate) copolymer, triethoxycaprylylsilane, and the like.

Examples of the silicone oil include methylhydrogen polysiloxane, dimethyl polysiloxane, methylphenyl polysiloxane.

Examples of the alkylsilane include methyltrimethoxysilane, ethyltrimethoxysilane, hexyltrimethoxysilane, octyltrimethoxysilane, and the like.

Examples of the fluoroalkylsilane include trifluoromethylethyltrimethoxysilane, heptadecafluorodecyltrimethoxysilane, and the like. In addition, the silicone compound may be a monomer of a compound or a copolymer thereof. One of these may be used alone, or two or more thereof may be used in combination.

As the fatty acid, for example, palmitic acid, isostearic acid, stearic acid, lauric acid, myristic acid, behenic acid, oleic acid, rosin acid, 12-hydroxystearic acid, and the like are mentioned.

As the fatty acid soap, for example, aluminum stearate, calcium stearate, aluminum 12-hydroxystearate, and the like are mentioned.

As the fatty acid ester, for example, dextrin fatty acid ester, cholesterol fatty acid ester, sucrose fatty acid ester, starch fatty acid ester, and the like are mentioned.

As the organic titanate compound, for example, isopropyl triisostearoyl titanate, isopropyl dimethacryl isostearoyl titanate, isopropyl tri(dodecyl) benzene sulfonyl titanate, neopentyl (diallyl)oxy-tri(dioctyl) phosphate titanate, neopentyl (diallyl)oxy-trineododecanoyl titanate, and the like are mentioned.

According to the titanium oxide powder of the present embodiment, in a case where the cosmetics containing the titanium oxide powder are applied to the skin, opacifying power is excellent. According to the titanium oxide powder of the present embodiment, in the case where cosmetics containing the titanium oxide powder are applied to the skin, it is possible to obtain natural finish in which paleness peculiar to titanium oxide particles is decreased and an excellent feeling of transparency is achieved in addition to an excellent opacifying power.

[Method for manufacturing titanium oxide powder]

A method for manufacturing a titanium oxide powder of the present embodiment preferably includes a first step of preparing a reaction solution by mixing a hydrolyzed product of a titanium alkoxide or a hydrolyzed product of a titanium metal salt with a compound having a five-membered ring that contains nitrogen, and subjecting this reaction solution to hydrothermal synthesis, to produce titanium oxide particles. In addition, the method for manufacturing a titanium oxide powder of the present embodiment includes a second step of drying the solution obtained in the first step at 400°C or lower.

(First step)

The first step is a step of producing titanium oxide particles.

In the first step, the hydrolyzed product of the titanium alkoxide or the hydrolyzed product of the titanium metal salt is mixed with the compound having the five-membered ring that contains nitrogen to prepare the reaction solution (slurry), and this reaction solution is subjected to hydrothermal synthesis to produce titanium oxide particles.

(Hydrolyzed product of titanium alkoxide or hydrolyzed product of titanium metal salt)

The hydrolyzed product of titanium alkoxide or the hydrolyzed product of titanium metal salt is obtained by hydrolyzing the titanium alkoxide or the titanium metal salt.

The hydrolyzed product is, for example, a cake-like solid which is a white solid, and is hydrated titanium oxide called metatitanic acid or orthotitanic acid.

As the titanium alkoxide, for example, tetraethoxytitanium, tetraisopropoxytitanium, tetra-n-propoxytitanium, tetra-n-butoxytitanium, and the like are mentioned. One of these may be used alone, or two or more thereof may be used in combination. Among these, tetraisopropoxytitanium and tetra-n-butoxytitanium are preferable, and tetraisopropoxytitanium is more preferable, from the viewpoint of easy availability and easy control of a hydrolysis rate.

As the titanium metal salt, for example, titanium tetrachloride, titanium sulfate, and the like are mentioned. One of these may be used alone, or two or more thereof may be used in combination.

In the present embodiment, in order to obtain highpurity anatase type titanium oxide particles, it is preferable to use a high purity titanium alkoxide or a high purity titanium metal salt.

The hydrolyzed product contains a by-product such as alcohols, hydrochloric acid, and sulfuric acid.

Since the by-product inhibits nucleation and crystal growth of titanium oxide particles, it is preferable to clean the hydrolyzed product with pure water.

Examples of a method for cleaning the hydrolyzed product include decantation, Nutsche method, ultrafiltration method, and the like.

(Compound having five-membered ring that contains nitrogen)

The compound having a five-membered ring that contains nitrogen is contained in the reaction solution due to a function as a pH adjuster of the reaction solution and a function as a catalyst for hydrothermal synthesis.

As the compound having a five-membered ring that contains nitrogen, for example, pyrrole, imidazole, indole, purine, pyrrolidine, pyrazole, triazole, tetrazole, isothiazole, isoxazole, furazan, carbazole, 1,5-diazabicyclo-[4.3.0]-5-nonene, and the like are mentioned. One of these may be used alone, or two or more thereof may be used in combination.

Among these, as the compound having a five-membered ring that contains nitrogen, a compound containing one nitrogen atom is preferable from the viewpoint of narrowing a particle size distribution of titanium oxide powder and of further improving crystallinity. For example, pyrrole, indole, pyrrolidine, isothiazole, isoxazole, furazan, carbazole, and 1,5-diazabicyclo-[4.3.0]-5-nonene are preferable.

Among these, as the compound having a five-membered ring that contains nitrogen, a compound which contains one nitrogen atom and of which a five-membered ring has a saturated heterocyclic structure is more preferable from the viewpoint of narrowing a particle size distribution of titanium oxide powder and of further improving crystallinity. For example, pyrrolidine, and 1,5-diazabicyclo-[4.3.0]-5-nonene are more preferable.

A method for preparing the reaction solution is not particularly limited, and can be appropriately selected depending on a purpose. For example, a method for mixing by using a stirrer, a bead mill, a ball mill, an attritor, a dissolver, or the like, and the like are mentioned.

In addition, water may be added to the reaction solution so that a concentration of the reaction solution is adjusted. As the water to be added to the reaction solution, deionized water, distilled water, pure water, and the like are mentioned.

The pH of the reaction solution is preferably 9 or more and 13 or less, and more preferably 11 or more and 13 or less, from the viewpoint that a catalytic action of the compound having a five-membered ring that contains nitrogen appropriately functions and a nucleation rate becomes appropriate.

In a case where the pH of the reaction solution is in a range of 9 or more and 13 or less, production of titanium oxide particles and efficiency of crystal growth become better.

The pH of the reaction solution can be regulated by controlling an amount of the compound having a five-membered ring that contains nitrogen.

A titanium atom concentration in the reaction solution can be appropriately selected depending on a size of each of the target titanium oxide particles, and the titanium atom concentration is preferably 0.05 mol/L or more and 3.0 mol/L or less, and more preferably 0.5 mol/L or more and 2.5 mol/L or less.

In a case where the titanium atom concentration in the reaction solution is 0.05 mol/L or more and 3.0 mol/L or less, a nucleation rate becomes appropriate, so that production of titanium oxide particles and efficiency of crystal growth become better.

The titanium atom concentration in the reaction solution can be regulated by controlling an amount of the hydrolyzed product of titanium alkoxide or the hydrolyzed product of titanium metal salt.

A molar ratio (titanium atom:compound having a five-membered ring that contains nitrogen) of titanium atoms to compounds having a five-membered ring that contains nitrogen in the reaction solution is preferably 1.0:0.5 to 1.0:2.0, and more preferably 1.0:0.6 to 1.0:1.8, and even more preferably 1.0:0.7 to 1.0:1.5.

In a case where the molar ratio of titanium atoms to compounds having a five-membered ring that contains nitrogen in the reaction solution is within the above-mentioned range, it is possible to produce octahedral-shaped titanium oxide particles.

Hydrothermal synthesis is a method in which a reaction solution is heated to allow titanium in the reaction solution to react in the presence of high-temperature and high-pressure hot water.

The hydrothermal synthesis is carried out by placing a reaction solution in a high-temperature and high-pressure container called an autoclave, sealing the autoclave, and heating the reaction solution together with the autoclave.

In a case where the reaction solution is heated, a pressure in the container rises due to evaporation of moisture in the reaction solution, which allows a high-temperature and high-pressure reaction to occur.

In the hydrothermal synthesis in the first step, octahedral-shaped titanium oxide particles are produced by holding the reaction solution at two different temperatures for a predetermined time. In the hydrothermal synthesis in the first step, the temperatures at which the reaction solution is held in a heated state are referred to as heating and holding temperatures. In addition, among the heating and holding temperatures, a low temperature is referred to as a first heating and holding temperature, and a high temperature is referred to as a second heating and holding temperature. Furthermore, the time for holding the first heating and holding temperature is referred to as a first heating and holding time, and the time for holding the second heating and holding temperature is referred to as a second heating and holding time.

The first heating and holding temperature in the hydrothermal synthesis is preferably 100°C or higher and 200°C or lower, and more preferably 120°C or higher and 180°C or lower.

By performing the second heating stage in the hydrothermal synthesis, the generation of particles and the growth reaction of particles can be separated from each other, and particles having a high degree of crystallinity can be obtained. That is, in the first heating stage, the temperature continues to increase. Therefore, it is considered that new fine particles are generated together with the growth of the generated fine particles, and coalescence of the fine particles that have not sufficiently grown occurs. The crystallinity of the particles generated by the coalescence of the fine particles is lowered due to the presence of interface misfit or the like between the fine particles. In a case where the first heating and holding temperature in the hydrothermal synthesis is within the above range, it is possible to prevent the titanium oxide fine particles from being excessively produced.

The first heating and holding time in the hydrothermal synthesis is preferably 1 hour or longer and 6 hours or shorter, and more preferably 2 hours or longer and 5 hours or shorter.

In a case where the first heating and holding time in the hydrothermal synthesis is within the above range, the produced titanium oxide fine particles can grow to consume the raw material, so that the generation of new fine particles during the second heating and holding can be prevented.

The second heating and holding temperature in the hydrothermal synthesis is preferably 200°C or higher and 350°C or lower, and more preferably 200°C or higher and 300°C or lower.

In a case where the second heating and holding temperature in the hydrothermal synthesis is within the above range, the growth reaction of the produced titanium oxide particles efficiently occurs.

The second heating and holding time in the hydrothermal synthesis is preferably 1 hour or longer and 24 hours or shorter, and more preferably 2 hours or longer and 12 hours or shorter.

In a case where the second heating and holding time in the hydrothermal synthesis is within the above range, the titanium oxide particles sufficiently grow and are excellent in production efficiency.

A heating rate in the hydrothermal synthesis is not particularly limited, and can be appropriately selected depending on a purpose.

A pressure in the hydrothermal synthesis is a pressure in a case where the reaction solution is heated to the above-mentioned temperature range in a high-temperature and high-pressure container.

During heating in the autoclave, it is preferable to stir the reaction solution using a stirring device.

A stirring speed is not particularly limited, and can be appropriately selected depending on a purpose. The stirring speed is preferably 100 rpm or more and 300 rpm or less.

(Second step)

A method for taking out the titanium oxide powder from the reaction solution after carrying out the first step is not particularly limited, and can be appropriately selected depending on a purpose. As the method for taking out the titanium oxide from the reaction solution, for example, a method for performing solid-liquid separation such as decantation and Nutsche method, and the like are mentioned.

After taking out the titanium oxide powder, the obtained titanium oxide powder may be cleaned with pure water or the like for the purpose of decreasing impurities.

The titanium oxide powder taken out by solid-liquid separation may be naturally dried, or may be heated at 400°C or lower and dried.

The lower limit of the heating temperature of the titanium oxide powder is not particularly limited as long as the titanium oxide powder can be dried. For example, the heating temperature may be 200°C, may be 250°C, or may be 300°C.

By drying the titanium oxide powder at 400°C or lower, it is possible to obtain a titanium oxide powder having excellent spreadability and adhesion to the skin in a case of being applied to the skin.

In a case where the drying temperature is high, the titanium oxide particles are fused with one another and the texture in a case of being applied to the skin is deteriorated, which is not preferable.

The titanium oxide powder of the present embodiment can be obtained by taking out the titanium oxide powder from the reaction solution which has been subjected to hydrothermal synthesis and drying the titanium oxide powder.

It is also possible to subject the titanium oxide powder to a surface treatment. A timing of performing the surface treatment is not particularly limited, and can be appropriately selected depending on a purpose. As the timing of performing the surface treatment, for example, after the second step, after the third step, and the like are mentioned.

A method for performing the surface treatment is not particularly limited, and a known method can be appropriately selected depending on a type of a surface treatment agent to be used.

[Dispersion]

The dispersion of the present embodiment contains the titanium oxide powder of the present embodiment and a dispersion medium. The dispersion of the present embodiment may contain other components as necessary.

The dispersion of the present embodiment may be in a low-viscosity liquid state or a high-viscosity paste state.

An amount of the titanium oxide powder in the dispersion of the present embodiment is not particularly limited, and can be appropriately selected depending on a purpose.

(Dispersion medium)

The dispersion medium is not particularly limited as long as the dispersion medium can be blended with cosmetics, and can be appropriately selected depending on a purpose. As the dispersion medium, for example, water, alcohols, esters, ethers, ketones, hydrocarbon, amides, polysiloxanes, modified polysiloxanes, hydrocarbon oil, ester oil, a higher fatty acid, higher alcohol, and the like are mentioned. One of these may be used alone, or two or more thereof may be used in combination.

As the alcohols, for example, methanol, ethanol, 1-propanol, 2-propanol, 1-butanol, 2-butanol, octanol, glycerin, and the like are mentioned.

As the esters, for example, ethyl acetate, butyl acetate, ethyl lactate, propylene glycol monomethyl ether acetate, propylene glycol monoethyl ether acetate, g-butyrolactone, and the like are mentioned.

As the ethers, for example, diethyl ether, ethylene glycol monomethyl ether (methyl cellosolve), ethylene glycol monoethyl ether (ethyl cellosolve), ethylene glycol monobutyl ether (butyl cellosolve), diethylene glycol monomethyl ether, diethylene glycol monoethyl ether, and the like are mentioned.

As the ketones, for example, acetone, methyl ethyl ketone, methyl isobutyl ketone, acetyl acetone, cyclohexanone, and the like are mentioned.

Examples of hydrocarbons include aromatic hydrocarbons and cyclic hydrocarbons.

Examples of aromatic hydrocarbons include benzene, toluene, xylene, ethylbenzene, and the like.

Examples of cyclic hydrocarbons include cyclohexane and the like.

As the amides, dimethylformamide, N,N-dimethylacetoacetamide, N-methylpyrrolidone, and the like are mentioned.

Examples of polysiloxanes include chain polysiloxanes, cyclic polysiloxanes, and the like.

Examples of chain polysiloxanes include dimethyl polysiloxanes, methylphenyl polysiloxanes, diphenyl polysiloxanes, and the like.

Examples of the cyclic polysiloxanes include octamethylcyclotetrasiloxanes, decamethylcyclopentasiloxanes, dodecamethylcyclohexasiloxanes, and the like.

As the modified polysiloxanes, for example, an amino-modified polysiloxane, a polyether-modified polysiloxane, an alkyl-modified polysiloxane, a fluorine-modified polysiloxane, and the like are mentioned.

As the hydrocarbon oil, for example, liquid paraffin, squalane, isoparaffin, branched chain light paraffin, petrolatum, ceresin, and the like are mentioned.

As the ester oil, for example, isopropyl myristate, cetyl isooctanoate, glyceryl trioctanoate, and the like are mentioned.

As the higher fatty acid, for example, lauric acid, myristic acid, palmitic acid, stearic acid, and the like are mentioned.

As the higher alcohol, for example, lauryl alcohol, cetyl alcohol, stearyl alcohol, hexyl dodecanol, isostearyl alcohol, and the like are mentioned.

(Other components)

The other components are not particularly limited as long as the components do not impair an effect of the dispersion of the present embodiment, and can be appropriately selected depending on a purpose. As the other components, for example, a dispersant, a stabilizer, a watersoluble binder, a thickener, an oil-soluble preservative, an ultraviolet absorber, an oil-soluble agent, oil-soluble coloring matters, oil-soluble proteins, a vegetable oil, an animal oil, and the like are mentioned. One of these may be used alone, or two or more thereof may be used in combination.

An amount of the dispersion medium is not particularly limited, and can be appropriately selected depending on a purpose. An amount of the dispersion medium is preferably 10% by mass or more and 99% by mass or less, more preferably 20% by mass or more and 90% by mass or less, and even more preferably 30% by mass or more and 80% by mass or less, with respect to a total amount of the dispersion of the present embodiment.

According to the dispersion of the present embodiment, in a case where the cosmetics containing the dispersion of the present embodiment are applied to the skin, opacifying power is excellent. According to the dispersion of the present embodiment, in the case where cosmetics containing the titanium oxide powder are applied to the skin, it is possible to obtain natural finish in which paleness peculiar to titanium oxide particles is decreased and an excellent feeling of transparency is achieved in addition to an excellent opacifying power.

[Method for manufacturing dispersion]

A method for manufacturing the dispersion of the present embodiment is not particularly limited, and a known method can be adopted. As the method for manufacturing the dispersion of the present embodiment, for example, a method for manufacturing a dispersion by mechanically dispersing the titanium oxide powder of the present embodiment with respect to a dispersion medium by a dispersing device, and the like are mentioned.

As the dispersing device, a stirrer, a self-revolution type mixer, a homomixer, an ultrasonic homogenizer, a sand mill, a ball mill, a roll mill, and the like are mentioned.

In a case of being applied to the skin, the dispersion of the present embodiment can decrease paleness peculiar to titanium oxide while achieving both the opacifying power and feeling of transparency. In addition, a feeling of squeaky is suppressed. Furthermore, in a case where the cosmetics containing the titanium oxide powder of the present embodiment are applied to the skin, spreadability and adhesion to the skin are excellent.

[Cosmetics]

The cosmetics of the present embodiment contain the titanium oxide powder of the present embodiment and a cosmetic base. The cosmetics of the present embodiment contain other components as necessary.

The amount of the titanium oxide powder in the cosmetics can be optionally selected, but the amount of the titanium oxide powder is preferably 0.1% by mass or more and 50% by mass or less, with respect to a total of the cosmetics.

(Cosmetic base)

The cosmetic base can be appropriately selected from generally used cosmetics. Examples thereof include talc, mica, and the like. One of these may be used alone, or two or more thereof may be used in combination.

An amount of the cosmetic base in the cosmetics is not particularly limited, and can be appropriately selected depending on a purpose.

(Other components)

In addition to the titanium oxide powder and the cosmetic base of the present embodiment, the cosmetics of the present embodiment can contain other components within a range which does not impair an effect of the present embodiment.

The other components can be appropriately selected from components generally used in cosmetics. As the other components, for example, a solvent, an oil agent, a surfactant, a humectant, an organic ultraviolet absorber, an antioxidant, a thickener, a fragrance, a colorant, a physiologically active component, an antibacterial agent, and the like are mentioned. One of these may be used alone, or two or more thereof may be used in combination.

An amount of the other components in the cosmetics is not particularly limited, and can be appropriately selected depending on a purpose.

A method for manufacturing the cosmetics of the present embodiment is not particularly limited, and can be appropriately selected depending on a purpose. As the method for manufacturing the cosmetics of the present embodiment, for example, a manufacturing method in which the titanium oxide powder is mixed with the cosmetic base and the mixture is mixed with the other components, a manufacturing method in which the titanium oxide powder is mixed with existing cosmetics, a manufacturing method in which the dispersion is mixed with the cosmetic base and the mixture is mixed with the other components, and a manufacturing method in which the dispersion is mixed with existing cosmetics, and the like are mentioned.

(Form)

A form of the cosmetics of the present embodiment is not particularly limited, and can be appropriately selected depending on a purpose. As the form of the cosmetics of the present embodiment, for example, a powder-like form, powdery solid-like form, a solid-like form, a liquid-like form, a gel-like form, and the like are mentioned. In a case where the form of the cosmetics is liquid-like or gel-like, a dispersion form of the cosmetics is not particularly limited, and can be appropriately selected depending on a purpose. As the dispersion form of the gel-like cosmetics, for example, a water-in-oil type (W/O type) emulsion, an oil-in-water type (O/W type) emulsion, an oil type, and the like are mentioned.

As the cosmetics of the present embodiment, for example, base makeup, nail polish, lipstick, and the like are mentioned. Among these, the base makeup is preferable.

As the base makeup, for example, makeup base used mainly for decreasing irregularities of the skin, foundation used mainly for adjusting a color of the skin, face powder used mainly for improving fixation of foundation to the skin, and the like are mentioned.

According to the cosmetics of the present embodiment, in a case of being applied to the skin, the opacifying power is excellent. According to the cosmetics of the present embodiment, it is possible to decrease paleness peculiar to titanium oxide particles while having a feeling of transparency.

Examples

Hereinafter, the present invention will be described more specifically with reference to Examples and Comparative Examples. However, the present invention is not limited to the following Examples.

[Example 1]

(Production of titanium oxide powder)

1 L of pure water was placed in a glass container having a capacity of 2 L, and 2 mol of tetraisopropoxytitanium (trade name: A-1, manufactured by Nippon Soda Co., Ltd.) was added dropwise while performing stirring to obtain a white suspension containing a hydrolyzed product of a titanium alkoxide.

Next, the white suspension was subjected to filtration, to obtain a white cake (A) that is a solid portion of the hydrolyzed product of titanium alkoxide.

Next, 2 mol (160 g) of the white cake (A) in terms of titanium oxide, pyrrolidine (manufactured by Kanto Chemical Co., Inc.) in an amount of 1.4 mol, and pure water were added to an autoclave to prepare a slurry (B1) in a total amount of 1 kg.

Next, by using the autoclave, after sealing the autoclave, the slurry (B1) was held at 150°C for 6 hours, then heated to 260°C, and held at the temperature of 260°C for 6 hours to obtain a titanium oxide particle suspension (C1) .

The obtained titanium oxide particle suspension (C1) was subjected to solid-liquid separation, and the solid was dried at 200°C to obtain a titanium oxide powder of Example 1.

(Production of cosmetics)

2 g of the titanium oxide powder of Example 1 and 8 g of talc were mixed with each other in a mortar to produce base makeup cosmetics of Example 1.

[Example 2]

(Production of titanium oxide powder)

A titanium oxide particle suspension (C2) was obtained in the same manner as in Example 1, except that by using the autoclave, the slurry (B1) was held at 170°C for 6 hours, then heated to 260°C, and held at the temperature of 260°C for 6 hours.

The obtained titanium oxide particle suspension (C2) was subjected to solid-liquid separation, and the solid was dried at 200°C to obtain a titanium oxide powder of Example 2.

(Production of cosmetics)

In the same manner as in Example 1, base makeup cosmetics of Example 2 were produced.

[Example 3]

(Production of titanium oxide powder)

A titanium oxide particle suspension (C3) was obtained in the same manner as in Example 1, except that by using the autoclave, after sealing the autoclave, the slurry (B1) was held at 130°C for 6 hours, then heated to 260°C, and held at the temperature of 260°C for 6 hours.

The obtained titanium oxide particle suspension (C3) was subjected to solid-liquid separation, and the solid was dried at 200°C to obtain a titanium oxide powder of Example 3.

(Production of cosmetics)

In the same manner as in Example 1, base makeup cosmetics of Example 3 were produced.

[Comparative Example 1]

(Production of titanium oxide powder)

1 L of pure water was placed in a glass container having a capacity of 2 L, and 1 mol of tetraisopropoxytitanium (trade name: A-1, manufactured by Nippon Soda Co., Ltd.) was added dropwise while performing stirring to obtain a white suspension containing a hydrolyzed product of a titanium alkoxide.

Next, the white suspension was subjected to filtration, to obtain a white cake (X) that is a solid portion of the hydrolyzed product of titanium alkoxide.

Next, pyrrolidine (manufactured by Kanto Chemical Co., Inc.) in an amount of 0.7 mol and 1 mol (80 g) of the white cake (X) in terms of titanium oxide were placed to an autoclave, and pure water was added therein to prepare a slurry (B11) in a total amount of 1 kg.

Next, by using the autoclave, after sealing the autoclave, the slurry (B11) was held at 220°C for 9 hours to obtain a reaction solution (C11) containing the titanium oxide particles.

Next, 100 g of the above white suspension (C11) (containing 8 g of titanium oxide), 1 mol (80 g) of the white cake (X) in terms of titanium oxide, pyrrolidine (manufactured by Kanto Chemical Co., Inc.) in an amount of 0.7 mol, and pure water were added to an autoclave to prepare a slurry (B12) in a total amount of 1 kg.

Next, by using the autoclave, after sealing the autoclave, the slurry (B12) was held at 220°C for 9 hours to obtain a titanium oxide particle suspension (C12).

The obtained titanium oxide particle suspension (C12) was subjected to solid-liquid separation, and the solid was dried at 200°C to obtain a titanium oxide powder of Comparative Example 1.

(Production of cosmetics)

In the same manner as in Example 1, base makeup cosmetics of Comparative Example 1 were produced.

[Comparative Example 2]

(Production of titanium oxide powder)

Next, 100 g of the reaction solution (C12) containing the titanium oxide particles that are obtained in the production process of Comparative Example 1 (containing 8.8 g of titanium oxide), 1 mol (80 g) of the white cake (X) in terms of titanium oxide, pyrrolidine (manufactured by Kanto Chemical Co., Inc.) in an amount of 0.7 mol, and pure water were added to an autoclave to prepare a slurry (B13) in a total amount of 1 kg.

Next, by using the autoclave, the slurry (B13) was held at 220°C for 9 hours to obtain a titanium oxide particle suspension (C13).

The obtained titanium oxide particle suspension (C13) was subjected to solid-liquid separation, and the solid was dried at 200°C to obtain a titanium oxide powder of Comparative Example 2.

(Production of cosmetics)

In the same manner as in Example 1, base makeup cosmetics of Comparative Example 2 were produced.

[Evaluation]

(Identification of shape of titanium oxide particle)

The titanium oxide powders obtained in Example 1 to Example 3, and Comparative Example 1 and Comparative Example 2 were observed with a transmission electron microscope (TEM) (model number: H-800, manufactured by Hitachi High-Technologies Corporation). From TEM images, it was confirmed that the titanium oxide particles constituting the titanium oxide powders obtained in Example 1 to Example 3, and Comparative Example 1 and Comparative Example 2 were octahedral-shaped particles.

(Measurement of maximum value and minimum value of line segments in one particle of titanium oxide particles, each of which connects two apexes facing each other)

Shapes of these titanium oxide particles were observed by observing secondary electron images of the titanium oxide powders obtained in Example 1 to Example 3, and Comparative Example 1 and Comparative Example 2 with a scanning electron microscope (SEM) (trade name: JSM-7200F, manufactured by JEOL Ltd.). By observing 100 titanium oxide particles in the secondary electron images, a maximum value ((X) shown in FIG. 2) of line segments in one particle, each of which connects two apexes facing each other, and a minimum value ((Y) shown in FIG. 2) of line segments, each of which connects two apexes facing each other and is orthogonal to the line segment corresponding to the maximum value, were measured with respect to the titanium oxide particles obtained in Example 1 to Example 3, and Comparative Example 1 and Comparative Example 2.

The obtained maximum value (X) was cubed and multiplied by a constant of 0.08 to calculate a volume of each titanium oxide particle. The volume particle diameter distribution was calculated using the maximum value (X) and the volume value thereof. The maximum value (X) (d50) of the obtained volume particle diameter distribution in a case where the cumulative volume percentage was 50% was calculated and used as an average value of the maximum values (X). The results are shown in Table 1.

In addition, an arithmetic mean value of the ratios of the maximum values (X) of titanium oxide particles to the minimum values (Y) of titanium oxide particles (maximum value (X)/minimum value (Y)) was calculated, respectively. The results are shown in Table 1.

(Measurement of amount of octahedral-shaped particle (% by number))

100 titanium oxide particles were observed using the secondary electron images of the titanium oxide powders obtained in Examples 1 to 3 and Comparative Examples 1 and 2 described above described above, and the octahedral-shaped titanium oxide particles included in the 100 particles were counted, so that an amount (% by number) of the octahedral-shaped particle was calculated. The results are shown in Table 1.

(Measurement of BET specific surface area)

A BET specific surface area of each of the titanium oxide powders obtained in Example 1 to Example 3, and Comparative Example 1 and Comparative Example 2 was measured using a specific surface area meter (trade name: BELSORP-mini, manufactured by Bel Japan, Inc.). The results are shown in Table 1.

(Average particle diameter converted from BET specific surface area)

An average particle diameter (BET-converted average particle diameter) converted from the BET specific surface area of each of the titanium oxide powders obtained in Example 1 to Example 3, and Comparative Example 1 and Comparative Example 2 was calculated by the following Expression (1) . The results are shown in Table 1.

$$\text{BET-converted average particle diameter (nm)} = 16240/(\text{BET specific surface area } (m^2/g) \times \rho \ (g/cm^3)) \ ... \ (1)$$

In Expression (1), ρ represents a density of the titanium oxide, and ρ = 4 g/cm$^3$.

A value obtained by dividing the average value of the maximum values (X) of the line segments in one particle of the titanium oxide particles, each of which connects two apexes facing each other, by the BET-converted average particle diameter (the average value of the maximum values (X)/the BET-converted average particle diameter) was calculated. The results are shown in Table 1.

(Measurement of crystallinity of titanium oxide powder)

A crystallinity of the titanium oxide powders obtained in Example 1 to Example 3, and Comparative Example 1 and Comparative Example 2 was measured as follows. X-ray intensity was measured in an output of 45 kV and 40 mA at a diffraction angle 2θ in a range of 20° to 30° using CuKα ray as an X-ray source with an X-ray diffractometer (trade name: X'Pert PRO MPS, manufactured by Malvern Panalytical Ltd.). The obtained X-ray diffraction pattern was profile-fitted for a crystalline portion (peak) and an amorphous portion (halo), and integrated intensities of each were calculated. Next, a ratio of the integrated intensities of the crystalline portion to a total integrated intensity was defined as a crystallinity. The results are shown in Table 1.

(Evaluation of opacifying power)

The base makeup cosmetics of Example 1 were applied on the entirety of one side of a 5 cm square substrate (trade name: HELIOPLATE HD-6, manufactured by Helioscreen) so as to be 12 mg to 14 mg to produce applied substrates.

A diffuse reflection spectrum (TR) of the coated substrate was measured using a spectrophotometer (model number: UV-3150, manufactured by Shimadzu Corporation) and evaluated using the following indices. A light reflection spectrum was measured on the basis of a molded plate obtained by compressing barium sulfate powder (special grade, manufactured by Kanto Chemical Co., Inc.).

The results are shown in Table 1.

The diffuse reflectance (TR$_{550nm}$) at 550 nm was used as an index for opacifying power. In a case where the diffuse reflectance is large, it can be said that the opacifying power is large. Thus, it is preferable that the value is large.

[Table 1]

| | Example 1 | Example 2 | Example 3 | Comparative Example 1 | Comparative Example 2 |
|---|---|---|---|---|---|
| Shape | Octahedron | Octahedron | Octahedron | Octahedron | Octahedron |
| BET specific surface area [m$^2$/g] | 9 | 9 | 8 | 9 | 6 |
| BET-converted average particle diameter [nm] | 451 | 451 | 507 | 451 | 677 |
| Average value of maximum values (X) [nm] | 470 | 450 | 550 | 450 | 740 |
| Maximum values (X)/(BET-converted average particle diameter) | 1.0 | 1.0 | 1.1 | 1.0 | 1.1 |
| Average value of maximum value (X)/minimum value (Y) | 2.0 | 2.0 | 2.0 | 2.0 | 2.0 |
| % by number | 65 | 65 | 65 | 65 | 60 |
| Crystallinity | 0.98 | 0.99 | 0.99 | 0.92 | 0.93 |
| Opacifying power (%) | 60 | 59 | 63 | 50 | 53 |
| First heating and holding temperature | 150 | 170 | 130 | 220 | 220 |
| First heating and holding time | 6 | 6 | 6 | 9 | 9 |
| Second heating and holding temperature | 260 | 260 | 260 | - | - |

(continued)

|  | Example 1 | Example 2 | Example 3 | Comparative Example 1 | Comparative Example 2 |
|---|---|---|---|---|---|
| Second heating and holding time | 6 | 6 | 6 | - | - |

By comparing Example 1 to Example 3 with Comparative Example 1 and Comparative Example 2, it was confirmed that the titanium oxide powders obtained in Example 1 to Example 3 having a crystallinity of 0.98 or higher have more excellent opacifying power than the titanium oxide powder having a crystallinity of 0.92 in Comparative Example 1 and the titanium oxide powder having a crystallinity of 0.93 in Comparative Example 2.

Industrial Applicability

The titanium oxide powder of the present invention contains polyhedral-shaped titanium oxide particles, in which each particle of the polyhedral-shaped titanium oxide particles has eight or more faces and an average primary particle diameter is 300 nm or higher and 1000 nm or lower, and a crystallinity is 0.95 or higher. Therefore, opacifying power is excellent in a case of being applied to the skin. Therefore, the titanium oxide powder can be suitably used for base makeup cosmetics such as foundations. Furthermore, since the titanium oxide powder of the present invention has excellent performance as a white pigment, the titanium oxide powder can be used for industrial applications such as white ink and is thus industrially valuable.

Reference Signs List

A, B, C, D, E, F: apex of octahedral-shaped particle a, b, c, d, e, f, g, h, i, j, k, 1: side (line segment) connecting two apexes or distance thereof
m, n, o: line (line segment) connecting two apexes inside particle, or distance
X: line segment having maximum distance between two apexes facing each other, or length
Y: line segment connecting two other apexes substantially orthogonal to line segment having maximum distance between two apexes facing each other, or its length (minimum value)

**Claims**

1.  A titanium oxide powder comprising:

    polyhedral-shaped titanium oxide particles,

       wherein each particle of the polyhedral-shaped titanium oxide particles has eight or more faces, and
       an average primary particle diameter is 300 nm or higher and 1000 nm or lower,

    wherein a crystallinity is 0.95 or higher.

2.  The titanium oxide powder according to claim 1, wherein the polyhedral-shaped titanium oxide particles each of which has eight or more faces comprise octahedral-shaped particles,

       wherein each particle of the octahedral-shaped particles has line segments each of which connects two apexes facing each other and has a maximum value of the line segments, and
       an average value of the maximum values is 300 nm or higher and 1000 nm or lower.

3.  The titanium oxide powder according to claim 1 or 2,
    wherein a BET specific surface area is 5 $m^2/g$ or higher and 15 $m^2/g$ or lower.

4.  The titanium oxide powder according to any one of claims 1 to 3,
    wherein a value obtained by dividing the average value of the maximum values by an average particle diameter converted from the BET specific surface area (the average value of the maximum values/a BET-converted average particle diameter) is 0.5 or higher and 2.5 or lower.

**5.** The titanium oxide powder according to any one of claims 1 to 4,
wherein an amount of the polyhedral-shaped titanium oxide particles, each of which has eight or more faces, in the titanium oxide powder is 50% by number or more.

**6.** The titanium oxide powder according to any one of claims 1 to 5,
wherein the titanium oxide powder has any one of an inorganic compound or an organic compound on a surface of the particle.

**7.** A dispersion, comprising:

the titanium oxide powder according to any one of claims 1 to 6; and
a dispersion medium.

**8.** A cosmetic comprising:

the titanium oxide powder according to any one of claims 1 to 6; and
a cosmetic base.

FIG. 1

FIG. 2

**INTERNATIONAL SEARCH REPORT**

| International application No. |
| --- |
| PCT/JP2019/034077 |

| A. CLASSIFICATION OF SUBJECT MATTER |
| --- |
| Int.Cl.  C01G23/04(2006.01)i, A61K8/29(2006.01)i, A61Q1/12(2006.01)i |

According to International Patent Classification (IPC) or to both national classification and IPC

| B. FIELDS SEARCHED |
| --- |
| Minimum documentation searched (classification system followed by classification symbols)<br>Int.Cl.  C01G23/04, A61K8/29, A61Q1/12 |

| Documentation searched other than minimum documentation to the extent that such documents are included in the fields searched | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

| C. DOCUMENTS CONSIDERED TO BE RELEVANT | | |
| --- | --- | --- |
| Category* | Citation of document, with indication, where appropriate, of the relevant passages | Relevant to claim No. |
| A | WO 2018/003851 A1 (SUMITOMO OSAKA CEMENT CO., LTD.) 04 January 2018 & US 2019/0161360 A1 & EP 3480167 A1 & CN 109476501 A & KR 10-2019-0021346 A | 1-8 |
| A | WO 2017/115802 A1 (SUMITOMO OSAKA CEMENT CO., LTD.) 06 July 2017 & US 2019/0016606 A1 & EP 3398909 A1 & CN 108430926 A & KR 10-2018-0097557 A | 1-8 |

☒ Further documents are listed in the continuation of Box C.  ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 06 November 2019 (06.11.2019) | 19 November 2019 (19.11.2019) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

**INTERNATIONAL SEARCH REPORT**

International application No.

PCT/JP2019/034077

C (Continuation). DOCUMENTS CONSIDERED TO BE RELEVANT

| Category* | Citation of document, with indication, where appropriate, of the relevant passages | Relevant to claim No. |
|---|---|---|
| A | JP 2009-521392 A (JOMA INTERNATIONAL AS) 04 June 2009 & US 2008/0299036 A1 & WO 2007/074436 A1 & KR 10-2008-0081938 A & CN 101346313 A | 1-8 |

Form PCT/ISA/210 (continuation of second sheet) (January 2015)

**EP 3 845 494 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018163676 A

- WO 2018003851 A